# EUROPEAN PATENT SPECIFICATION

(11) **EP 3 655 959 B1**
(45) Date of publication and mention of the grant of the patent: **27.09.2023**
(21) Application number: 18732936.2
(22) Date of filing: 07.06.2018
(51) Int. Cl.: G06Q 10/10, G09B 19/00, G16H 20/40, G16H 30/40, G16H 50/30, G16H 50/20

(54) **METHOD AND SYSTEM FOR PROVIDING A UNIQUELY MAPPED DENTAL HEALTH SERVICE PACKAGE**
VERFAHREN UND SYSTEM ZUR BEREITSTELLUNG EINES EINDEUTIG DARGESTELLTEN ZAHNGESUNDHEITSDIENSTPAKETS
PROCÉDÉ ET SYSTÈME POUR FOURNIR UN ENSEMBLE DE SERVICES DE SANTÉ DENTAIRE À MAPPAGE UNIQUE

(30) Priority: 19.07.2017 NL 2019274
(43) Date of publication of application: 27.05.2020
(73) Proprietor: Gründemann, Lodewijk Johannes Michael Maria, 9084 AG Goutum (NL); Vroom, Melle Gerardus, 9084 AG Goutum (NL)
(72) Inventor: Gründemann, Lodewijk Johannes Michael Maria, 9084 AG Goutum (NL); Vroom, Melle Gerardus, 9084 AG Goutum (NL)
(74) Representative: Arnold & Siedsma
(86) International application number: PCT/NL2018/050368
(87) International publication number: WO 2019/017768

(56) References cited:
- EP-A2- 2 384 720
- EP-A2- 2 384 720
- Juli Clover: "Hands-On Review of Oral-B's iPhone-Connected Bluetooth Smart Toothbrush - Mac Rumors", , 6 February 2015 (2015-02-06), pages 2-15, XP055400026, Retrieved from the Internet: URL:https://www.macrumors.com/2015/02/06/o ral-b-pro-smart-toothbrush-review/ [retrieved on 2017-08-21]

## Description

The invention relates to a method and a system for providing a uniquely mapped dental health service package.

It is generally accepted that good dental care is required to obtain a healthy set of teeth. Such dental care includes regular visits to a dental specialist, such as a dentist, regular dental care performed by a person in daily life, such as brushing the teeth and interdental cleaning and, in some cases, a balanced nutrition. However, brushing and interdental cleaning is only useful when it is performed using a correct brushing and/or interdental cleaning technique. Also, the use of correct brushing and/or interdental cleaning equipment forms an important factor in the effectiveness of brushing and interdental cleaning.

Additionally, it is important that the dental care actions performed by a user are adapted to the unique set of teeth of the user in that the dental care actions are adapted to the lay-out and condition of the set of teeth, including missing teeth, specific medical conditions etc.

Although most dental specialists during an appointment provide a person with instructions and information that are specifically adapted to the unique set of teeth of the user, it is often (in up to 80% of the cases) incorrectly remembered or even (completely) forgotten by the person at the time, for example during his cleaning session, when it needs to be applied. As a result, the person performs incorrect dental care actions or forgets to perform all instructed dental care actions, leading to insufficient cleaning to obtain a good dental health.

The dental specialist is on the other hand limited in his actions in that he can only provide instructions and/or dental care actions adapted to the person during a physical meeting with the person (in this case his patient), since he is not present when the instructions and/or dental care actions are to be applied or performed on a daily basis.

The technical problem can therefore be defined as how to provide a user with detailed information and/or dental care actions specifically adapted to his set of teeth at the moment of use of that information and/or at the moment the dental care actions are to be performed by the user.

The method according to the invention obviates or at least reduces the abovementioned problem.

To that end, the invention provides a method according to claim 1.

It should be noted that, for the purposes of the invention, the dental scheme should be considered to include and/or be equivalent to a dental hygiene scheme and/or a dental care performance scheme and/or a dental care scheme.

Providing a uniquely mapped dental health service package to a user provides several advantages. First of all, the uniquely mapped dental health service package allows a user direct access to a (detailed) dental scheme including information on the correct brushing and/or interdental cleaning technique that is provided by the user's dental specialist. In other words, the dental scheme provides an overview of all teeth physically present in the user's set of teeth and the dental care actions that are to be performed associated with that specific lay-out of teeth. This for example also means that, if a tooth is missing (i.e. a gap is present in the set of teeth), the dental care action is adapted to the presence of the gap, for example by describing that the teeth adjacent to the gap need to be brushed on the edges adjacent to the gap. The result of associating the dental care actions to be performed with the actual lay-out of the teeth of the user, is that the user is provided with unique and optimized dental care for his set of teeth. This in turn is a direct result of the fact that the information in the dental scheme is adapted to the set of teeth of the user, and the user is therefore only provided with information that is relevant for that user.

As such, the uniquely mapped dental health service package according to the invention differs from existing brushing guides in that existing brushing guides only represent a general, non-adapted or 'standard' set of teeth and, as a consequence, a general 'standard' guideline for dental care actions to be performed. For instance on the Internet at URL:https:// www.macrumors.com/2015/02/06/oral-b-pro-smart-toothbrush-review/. The use of an existing brushing guide, which is not adapted to the specific set of teeth of the user, actually increases that insufficient and/or incorrect dental care actions are performed by the user, leading to reduced dental health.

The dental health service package therewith provides a significant improvement over existing brushing guides that can be found on internet.

The uniquely mapped dental health service package is provided by means of displaying the information on a screen or display of the digital device to the user.

Another advantage of the dental health service package according to the invention is that it supports a user by adapting his brushing and/or interdental cleaning behaviour into the behaviour recommended by the dental specialist for that specific user's set of teeth. A change in the dental care behaviour of the user to a behaviour adapted to the user reduces or prevents decay of the set of teeth of the user and/or gum diseases (gingivitis/periodontitis/mucositis/peri-implantitis) and additionally results in an improved health of the mouth area in general.

Yet another advantage of the dental health service package it that it may comprise additional features that are added to the dental health service package to achieve an even more extensive change in behaviour. Such additional features may include providing an interactive brushing and/or interdental cleaning guide to the user and/or incorporating a nutrition history coupled to a dental awareness and guidance program aimed at improving dental health of the user by means of providing uniquely mapped and personalized information.

Another advantage of the dental health service package is that it is independent from suppliers of dental health equipment and can be used in conjunction with equipment from any manufacturer. This is not possible using commercial apps, such a simple brushing apps. As a result, the dental health service package provides a better experience and allows dental specialists and users to use the dental health service package to assist in the dental health care and, preferably, also allows them to communicate on the subject on the set of teeth of the user. This means the dental health service package supersedes known (basic) brushing apps and provides a detailed, personalised portal for high-end dental health care that is currently not yet available.

The dental health service package is configured such that the user can at any time access the information that is uniquely mapped to that user for performing dental care actions for his set of teeth. To that end, the dental health service package may be stored in a central memory, which is accessible using a digital device, which preferably is a digital device of the user, for example with a dental health service package application. This direct link from the digital device of the user to the memory is preferred to establish a relatively privacy-safe connection. It is however possible that one or more third parties are involved in accessing the memory and/or transferring the data therefrom to an end-user. The memory may however also comprise a local memory of a digital device of the user, such as a flash drive in a mobile phone, which is accessible from the digital device of the user.

In an embodiment according to the invention, the method of providing the uniquely mapped health service package to the user also comprises displaying on the digital device a representation, preferably a visualisation, of the risk profile of the user, wherein the risk profile comprises a user-specific mark that is compared with a, preferably scientifically known, benchmark, wherein the risk profile may comprise one or more of the plaque-index, the DPSI-index, the smoking index and/or the bleeding index.

The dental health service package is configured to provide user-specific dental health information and guidance that would otherwise not be available for the patient at the time it is required (for example when performing dental health actions). In addition, the dental health service package may be configured to support and motivate the user to improve the dental health of his/her set of teeth. This can for example be performed by calculating and/or displaying the risk profile of a user compared to a scientifically known benchmark. For example, the dental health service may be configured to display to the user a smoking index benchmark and his or her score on the smoking index benchmark that allows the user to determine/see his score compared to the average. This provides for a more comprehensive and inclusive dental health service aimed at improving dental health of the user.

In an embodiment according to the invention, the method additionally may comprise the step of authenticating the user using the digital device as being the user that is associated with the uniquely mapped dental health service package of that user, wherein authenticating comprises providing an access request including authentication information by the digital device to an authentication component associated with the memory, comparing authentication information in the access request to authentication information contained in the dental health service package and/or the memory and/or the authentication component, and, when the authentication information matches, allowing the digital device to access the memory, and when the authentication information does not match, denying the digital device to access the memory.

Since the uniquely mapped dental health service package contains medical information, it is preferable, and in some countries imperative, that the service package is only accessible by users that are allowed to view the information contained therein. Therefore, the method according to the invention additionally may comprise an authentication step for authenticating that a user is entitled to view the information. The authentication step may for example comprise using the digital device to provide a password or a biometric scan to the authentication component and subsequently comparing the password or biometric scan with a list of respectively passwords or biometric scans to authenticate whether access is allowed.

Yet another advantage of the dental health service package is that, in addition to providing a dental health scheme for the user, it may also comprise dental information on the modifications that are performed on the set of teeth. This may comprise for example (detailed) information on implants or crowns of teeth that have been placed, such as manufacturer, type, year of production and materials used. Furthermore, the information may also comprise an X-ray picture of an implant and/or the associated bone. Providing this information is beneficial for both the user and the dental specialist, since it provides direct access and history of the dental modifications that are performed on the set of teeth of the user.

The dental health service package comprises dental information on modifications, and it may also comprise an emergency function that allows a user to contact a dental specialist when experiencing dental health problems.

This provides the advantage that a user can provide detailed information on specific modifications made to his set of teeth to a dental specialist that is not his regular specialist. This is for example useful when a dental problem occurs during travel and the user visits a different dental specialist. In an elaboration, the dental health service package may even provide the user with a pointer or contact information of a nearby dental specialist that also makes use of the dental health service package software and/or system, which specialist can be contacted in case of emergency.

In an embodiment according to the invention, the method may comprise creating a dental health service package for an individual user that comprises performing at least one of a visual inspection by a dental specialist of the set of teeth of the user, creating and analysing a dental image of a set of teeth of the user, and, based on the analysis of the dental image and/or the visual inspection, creating the dental hygiene scheme by selecting dental care actions to be performed by the user on their set of teeth. The embodiment also includes adding personal information of the user to the dental health service package for identifying the user.

The basis for providing a uniquely mapped dental health service package is formed by creating and analysing a dental image of the set of teeth of the user. Preferably, the dental image is made by a dental specialist after or in addition to a visual inspection of the set of teeth of the user. The dental specialist subsequently analyses the dental image and creates a dental scheme by selecting dental care actions to the dental image. The dental care actions for the dental health service package are preferably provided by the dental specialist on the basis of his expertise in combination with the visual inspection and/or the dental image. The dental care actions represent codified knowledge of the dental specialist with regard to the dental care for that particular set of teeth. Preferably, the dental scheme is during an appointment at the dental specialist also directly communicated with the user of the device, which increases the awareness of the user with regard to the dental scheme. The dental health service package is further personalised or coupled to a user by adding additional personal information of the user to the package. The advantage of performing these method steps according to the invention is that the dental health service package is uniquely mapped to the user and has a scientific basis provided by the users dental specialist, therewith assuring a good dental health care for the user.

Furthermore, selecting the dental care actions may be combined with also selecting the correct products for the user that the user requires for preforming the selected dental care actions.

In an embodiment of the invention, analysing the dental image may comprise one or more of the following actions: determining a number of teeth and/or molars in the set of teeth of the user, determining a position of the teeth and/or molars in a jaw bone of the user and/or a position of the teeth and/or molars relative to each other, determining a position of any gaps or openings due to missing and/or extracted teeth and/or molars in a jaw bone of the user and/or a position of the gaps or openings due to missing and/or extracted teeth and/or molars relative to other teeth, and/or determining a state of dental care of the set of teeth of the user, including gums, dental nerves and/or dental roots associated with the set of teeth, the dental care state comprising determining the presence of one or more of dental plaque, crowns of a tooth, dental prostheses, oral implants, dental fillings and gingivitis, periodontitis, which may include appointing or calculating a Dutch Periodontal Screening Index score (or DPSI).

In an advantageous embodiment of the method according to the invention, analysing of the dental image comprises one or more actions performed by a dental specialist to configure the dental health service package. The analysis may be performed solely based on the dental image or may be based on a combination of the dental image and performing a visual inspection of the set of teeth of the user by the dental specialist. The analysis may comprise determining the number and location of teeth, molars and/or gaps caused by missing teeth and/or molars, which may for example influence the sequence, intensity and direction of brushing and/or interdental cleaning. Furthermore, the analysing advantageously comprises determining the state of dental care, which allows an even more detailed, and unique mapping of the dental health service package to a user.

In addition, the step of analysing the dental image may comprise obtaining, based on the dental image and information from research of the set of teeth by the dental specialist, specific characteristics of the teeth and/or molars, such as density, porosity, dental enamel and/or integral strength.

Additionally or alternatively, the step of analysing the dental image may also comprise interacting with the user of the dental health service package based on the dental image to obtain more specific information on the daily dental care actions that are performed by the user and/or specific problems with regard to the set of teeth of the user. The acquired information may be added to the dental image.

As a result, the expert knowledge of the dental specialist, preferably in combination with the knowledge and/or daily experiences from the user, is codified in dental health service package and forms the specific basis for selecting dental care actions.

In an embodiment according to the invention, the method may comprise the steps of retrieving the state of dental care of the set of teeth of the user in the dental health care service package, and retrieving a benchmark dental care state, wherein the benchmark dental care state is preferably stored in the memory, and comparing the dental care state of the set of teeth with the benchmark dental care state, and providing output to the user representing the results of the comparison, wherein the result preferably comprises an indication of whether the dental care state of the set of teeth of the user is worse, equal or better than the benchmark dental care state.

The dental health service package may advantageously be configured to, in addition to providing specific information on the dental care state of the users set of teeth, also provide a comparison between a dental state benchmark and the dental care state of the users set of teeth. This allows a user to measure the status and effect of his actions on the dental care state of his set of teeth. By comparing the dental care state of the specific (thus unique) set of teeth of the user with a benchmark, a personalised and traceable dental care history compared to the benchmark can be made. This allows the user to track whether the actions have result (in terms of dental health care) over time. It also provides a user with an incentive to improve (with a negative outcome) or maintain (equal or better outcome). The comparison may be made on one or more of different benchmarks, which may include scientifically known benchmarks such as the plaque-index, the DPSI-index, the smoking index (non-smoking, light smoker, heavy smoker) and/or the bleeding index (indicating the amount of bleeding during dental examination), wherein a healthy set of teeth does not exceed the benchmark value for the chosen benchmark(s). It is noted that (amongst dental specialists) the bleeding index is generally associated with the amount of bleeding during dental examination (for example dental (bleeding) examination score) and is determined by measuring, using a measuring instrument, the amount of bleeding at specific locations for each tooth and molar in the set of teeth, wherein the amount of bleeding provides the bleeding index.

This embodiment may also be used in conjunction with special steps and/or programs within the dental health service package aimed at juvenile users.

In an embodiment according to the invention, creating the dental image may comprise using the information, obtained from at least one of the actions regarding analysing the dental image as provided above, to create a digital image of the set of teeth of the user, wherein the digital image is unique for the set of teeth of the user, and providing the digital image to the dental health service package, and preparing the digital image by the dental health service package for adding information including dental care actions to be performed on the set of teeth of the individual user by the user, the preparing preferably comprising adding to the digital image one or more layers that are configured for containing and/or displaying information including dental care actions to be performed.

Creating the dental image is performed using information that is specific for that user in order to obtain a uniquely mapped dental image (and thus dental health service package. The information used in creating the dental image preferably includes the layout of the teeth and/or molars in the users set of teeth and (consequently) information on gaps between the teeth and/or molars. The dental image is added to the dental health service package and prepared for the addition of further information. Preferably, such preparations include adding one or more layers that are configured to contain specific information, such as information on the condition of a tooth or molar and/or specific characteristics of a modification (such a filling) that is made to the tooth and/or molar.

In an embodiment of the invention, the step of selecting dental care actions to be performed by the user may comprise storing first information comprising the sequence of brushing of the teeth of the user and storing second information comprising the duration and/or intensity of brushing to be performed by the user for each of teeth and/or molars, wherein the first and second information is uniquely mapped for the user.

Providing optimal dental health care for a user by the daily dental care actions requires the actions to be uniquely mapped for that specific user. Such unique mapping preferably comprises, based on the analysis of the dental image, providing a brushing and/or cleaning scheme containing dental care actions that, when applied by the user, provides an optimal dental care for that specific user. The brushing scheme is preferably based on the analysis of the dental image and takes into account the information contained therein. Preferably, the dental care actions are provided to the user on the digital device in the form of a visualisation, such as an image or a movie. Such a visualisation may also comprise an interactive visualisation that is configured to provide feedback to the user when the user provides user information to the digital device.

In an embodiment of the invention, the step of selecting dental care actions to be performed by the user may comprises storing third information comprising the sequence of interdental cleaning of the teeth of the user and storing fourth information comprising the duration and/or intensity of interdental cleaning to be performed by the user for each of the spaces between the teeth, wherein the interdental cleaning information is uniquely mapped for the user.

Interdental cleaning may in this application be understood as comprising flossing, interdental tooth picking using a toothpick, interdental brushing or other interdental actions aimed at cleaning the interdental spaces.

For most users, the optimal dental care is only achieved using a combination of brushing and interdental cleaning. The dental health service package therefore preferably incorporates dental care actions with regard to interdental cleaning that are uniquely mapped to the user. The necessity of incorporating interdental cleaning instructions in the dental health care service package are determined by the dental specialist on the basis of the dental image and/or visual inspection and/or information provided by the user.

It should be noted that the selection of the dental care actions, for example by means of the first, second, third and fourth information is preferably based on a combination of the expertise of the dental specialist that is applied to the set of teeth of the user. The dental care actions that are selected and supplied to the dental health service package are adapted to the specific user and, preferably, based on the knowledge of the dental specialist. It is conceivable that, especially using machine learning, the expertise of the dental specialist may be combined with computer-selected dental care actions based on the dental image.

According to the invention at least one of the first and second stored information, and optionally one or more of the third and fourth stored information, is provided to the user on a screen of the digital device, wherein providing the information comprises displaying the dental image of the user and a visual indicator that displays one or more of the sequence, duration and/or intensity of the brushing for each of the teeth, and optionally displays one or more of the interdental cleaning, preferably the interdental cleaning of the spaces between the teeth.

Although various different approaches can be used to provide the information in the dental health service package to the user, the approach according to the invention is to display the dental image of the user supplemented by a visual indicator that guides the user through the necessary dental care actions that need to be performed. Providing such an approach has the advantage that combines the unique mapping of the dental health service package with a visualisation of the sequence and content of the different dental care actions to be taken, therewith forming a uniquely mapped brushing movie.

In an embodiment of the invention, the dental image and the visual indicator may be incorporated in a video feed that is uniquely mapped to the user, such as a brushing movie, that provides a uniquely mapped guideline for dental care of the set of teeth of the user.

The advantage of a brushing movie according to this embodiment is that it provides all the advantages of a 'regular' brushing movie, while also having a scientific, specialist basis that is provided by the dental image analysis executed by the dental specialist that uniquely maps the required dental care actions for the specific user to obtain an optimal dental health care.

In an embodiment of the invention, the method may further comprise providing information on dental corrections and dental repairs, which include information on crowns of teeth, dental fillings, oral implants and dental prosthesis, preferably during visual inspection of the set of teeth of the user by the dental specialist, and adding the information on dental corrections and dental repairs to a dental image of the set of teeth of the user, preferably in the form of a digital, interactive layer on the dental image.

Aside from providing a dental hygiene scheme for a user, the dental health service package may also include a detailed mapping on dental corrections and/or dental repairs that have been performed over time. The detailed mapping can be added to the dental image, preferably by means of an interactive layer that is easily adaptable by a user and/or the dental specialist of the user. This information may include the type of correction or repair that has been performed and/or the techniques and/or materials used in the correction or repair. It may for example include the composition of materials, the manufacturer of dental prosthesis and/or the type and/or model of such dental prosthesis. It may also include any other information that the dental specialist may consider to be useful for the user and/or other people that may be given access to the uniquely mapped dental health service package of the user.

In an embodiment of the invention, the method further may comprise providing a dental emergency service, the service including contacting a dental emergency specialist or dental emergency institution by the user using a communication module of the uniquely mapped dental health service package, accessing the information in the uniquely mapped dental health service package. The method according to claim 1 comprises sending uniquely mapped information from the uniquely mapped dental health service package to (preferably a digital device of) the dental emergency service to allow the dental emergency service to render assistance to the user based on the dental information in the uniquely mapped dental health service package. The method according to claim 1 alternatively comprises authorising the dental emergency service to access the uniquely mapped dental health service package. This may include allowing a digital device of the dental emergency service to access the uniquely mapped dental health service package of the user to render assistance to the user based on the dental information in the uniquely mapped dental health service package.

The information contained in the dental health service package may advantageously used in case of (dental) emergencies in which the (regular) dental specialist of the user is not available by providing a dental emergency service to the user. The advantage of such a service is that, using the uniquely to the user mapped dental information of the user contained in or accessible by the dental health service package, it allows a dental specialist or dental institution providing dental emergency service to access or obtain all relevant dental information of the user. As a result, a more detailed and fact-based treatment or solution to the (dental) emergency of the user can be provided.

The dental health service package may be configured to provide a direct link to an available dental specialist or may provide a telephone number to be contacted to be directed to a dental specialist that is available instead of the regular dental specialist of the user.

According to claim 1, the dental health service package is, in one alternative, configured to allow the user to send some or all information on its teeth to the dental specialist or the dental institution that is to perform the dental emergency assistance. The transfer of the information may be provided over a communication network, but may also be provided by making a (cabled) connection between the digital device of the user and a digital device of the dental specialist or dental institution. This option allows the user to exercise ultimate control over his data to determine which data is provided to third parties. The information may comprise a screen shot of the dental image and/or a screen shot of the information on the tooth/molar that is currently causing the emergency. According to claim 1, the dental health service package in the other alternative is configured to allow the user to authorize a dental specialist or a dental institution access to the uniquely mapped dental health service package of the user. This allows a digital device of the dental specialist or the dental institution access to the information, therewith making it possible to provide tailored emergency assistance. Several options for providing such access are possible. This may comprise providing a digital notification or authorisation of the user towards the system for allowing access to that particular dental specialist. It may also comprise sending an access request or code, such as a QR code, to the digital device of the user, wherein the user than authorises the dental specialist by scanning or accepting the code. Other options for providing remote access may however also be used. According to claim 1, both alternatives may also be provided simultaneously.

In an embodiment of the invention, the method additionally may comprise providing a list of equipment that is uniquely mapped to the user and that may be required for performing the dental care actions in the dental scheme, wherein the list preferably is provided by a logistics module of the dental health service package.

In order to perform the dental care actions, a user often requires equipment, such as a toothbrush, interdental cleaning, tong cleaning and/or rinsing equipment. In an advantageous embodiment of the method according to the invention, the dental health service package is configured to provide the user with a list of equipment that is required for performing the dental care actions contained in the dental health service package. The dental specialist, preferably when creating the dental scheme, also compiles a list of required equipment for the dental care actions, which list is provided to the dental health service package. In this way, the dental specialist can in an easy manner provide the user with information on the required equipment, which the user can at any time review. Preferably, the list provides a detailed specification of the required equipment, which may include hardness and/or size, such as the diameter and hardness of an interdental brush.

In an embodiment, the uniquely mapped list of equipment may include an inventory module that is configured to allow the user to keep track of his personal equipment inventory for the dental service health package. This way, the user, preferably when accessing the dental health service package, is provided with insight in the status of the required equipment. It may also be possible to provide a notification to the user that the equipment on the list is running low, therewith allowing the user to timely order or purchase additional equipment. The dental health service package may therewith be a complete dental health support package. In an elaboration of the embodiment, the logistics module and/or the list may even contain links to websites and/or webshops that have the equipment mentioned in the list of equipment to allow a user to resupply his stock of required equipment without having to search for shops or sellers that have that are able to provide that specific equipment.

In an embodiment of the method according to the invention, the method additionally may comprise supplying to the dental health information package by the user one or more of instructions on dental health actions to be performed based on user-actions comprising intake of nutrition, information on nutrition intake of the user, such as sugar intake and feedback information to the dental health information package in response to a dental health actions performed by the user, and storing the respectively supplied instructions, information on nutrition intake, and/or feedback information in a logbook of the dental health service package. The instruction may also include information on the products that are required to perform the dental care actions in the dental health service package, which may be specific products that are adapted to the set of teeth of the user.

By providing a logbook module in which, the log is configured for entering, storing and/or retrieving information by the user and/or a dental specialist associated with the dental health service package of the user, the progress of and compliance with the dental care actions can be monitored. Supplying the actions may comprise confirming or entering data on the dental actions that he or she has performed, for example in a checklist of actions to be performed which can be checked of by the user. It may also comprise a more elaborate function in which the user can check off the actions to be performed and in addition add comments and/or questions with regard to the dental actions. The logbook function may be coupled to a communication module for sending and receiving, such as updates and/or advice of the dental specialist and/or questions and answers.

According to claim 1, the method comprises sending uniquely mapped push-notifications to the digital device of the user as part of the dental scheme, wherein the push-notifications include a notification of a dental hygiene action to be performed by the user according to the dental service package health. Optionally, the push-notifications may include one or more of the following notifications: a reminder of an appointment with the dental specialist, and a notification to fill out a questionnaire and/or update a status of the dental scheme actions to be performed by the user, such as providing information on the performed dental actions or the dental actions to be performed, wherein the questionnaire and/or the status update is coupled to the dental health service package and therewith is uniquely mapped to that user, and a feedback notification, preferably a random feedback notification, which notification is adapted to the information provided by the user in the status update or questionnaire.

The addition of push-notifications may assist users in complying with the dental hygiene scheme during the day. This is most useful for specific groups of users that would otherwise forget to perform the required actions.

In an embodiment of the method according to the invention, which comprises sending notifications and/or supplying information, the method may comprise providing an integrated dental awareness and guidance schedule that is uniquely mapped to the user, the user preferably being a child or young adult having an age of 18 years or less, the schedule comprising the steps of, based on the dental image and/or visual inspection of the set of teeth of the user, providing a risk analysis on the sensitivity of the set of teeth for tooth decay and/or gum diseases such as gingivitis and/or periodontitis, and drafting a guidance schedule for the user, the guidance schedule including the dental health actions to be performed and a date and frequency of feedback moments, and performing the steps of supplying information, and performing the step of sending notifications.

The dental health service package may comprise a dental awareness and guidance program that is preferably specifically tailored for children or young adults to teach them a proper dental care behaviour. As such, the dental awareness and guidance schedule contains information provided by the user, such as information on the nutrition history of the user, and information on actions to be performed as well as information on the progress concerning these actions. The user is in this way guided by the dental specialist by means of the dental awareness and guidance program in the dental health service package, which provides the user with a more specific insight in the dental care actions to be taken as well as the effects and results of the user's actions with regard to dental health. To support the user during the schedule, the dental health service package comprises sending push-notifications to the user which provide reminders for actions or queries into the progress of the schedule. The notifications may also include a reaction to the user that is tailored to the progress, such as a positive notification when the user complies to the schedule and an encouraging message to support the user's resolve in performing the actions.

The dental awareness and guidance program may also include a list of products that, based on the visual inspection of the set of teeth and/or the dental image and/or the experience of the dental specialist are required for performing the actions in the dental awareness and guidance program.

In an embodiment, the step of creating the dental image may comprise taking an X-ray picture of the set of teeth of the user and subsequently adapting the picture by adding characteristics of the set of teeth or providing a status praesens which is manually or automatically adapted by using visual inspection by the dental specialist, automatic image recognition techniques or combination thereof.

One of the advantages of the logbook is that the dental specialist may be provided with a more detailed insight in the dental actions performed by the user and/or the problems and/or questions that the user comes across during performing the actions. As a result, the dental specialist is also to provide a more detailed advice and/or diagnosis to the user.

The invention also relates to a system for providing a uniquely mapped dental health service package to a user according to claim 1.

The system according to the invention provides the same effects and advantages as the method according to the invention as described above. The system according to the invention first of all provides the advantage that a user has direct access to a dental hygiene scheme including information on the correct brushing and/or interdental cleaning technique that is provided by the user's dental specialist. Another advantage of the system according to the invention is that several options are available for configuring the system to provide the uniquely mapped dental health service package to the user.

The system may be configured as a stand-alone system in which a digital device of a user, such as a smart phone, forms the system on which the dental health service package is provided. In this system, each user has access to his or her own uniquely mapped dental health service package without requiring a connection with the internet or a communication network. This configuration has the advantage that it is useable in regions or locations in which access to internet and/or communication networks is limited or unavailable. Furthermore, it provides a user with increased control over his or her data, because the user can determine who can access the system on the digital device. It is preferred that the dental health service package is provided with authentication in case the digital device is lost or stolen. Also, by providing the system on the digital device of the user, the user is able to access the uniquely mapped dental health service package to retrieve the necessary data in case of emergency. The dental specialist of the user can, for example during a routine appointment, upload the dental health service package to the system that is formed by the digital device of the user.

The system may also be a system in which the dental health service packages are stored in a central memory, which is accessible for users and/or (authorised) dental specialists by using a digital device such as a smart phone. This configuration of the system can use a central memory, for example a cloud-based memory or a server park, in which a plurality of dental health service packages of users is stored. Each user can access the system from a digital device using an authentication code, wherein the authentication code is associated with his or her dental health service package. A dental specialist may have an authentication code that allows access to multiple dental health service packages of users, in which the users for example form his client group. The advantage of this system is that each user can access the dental health service package from a variety of different digital devices. The system may be configured to also allow the dental health service package to be at least partially downloaded to a digital device of a user.

According to the invention, the at least one processor of the system is configured for executing the method according to the invention.

In an embodiment of the system according to the invention, the system may additionally comprise a logistics component that is configured for storing a list of equipment required for performing the dental hygiene scheme of a dental health service package of the user and a user interface for allowing the user or the dental specialist associated with a dental health service package to modify the list of equipment that is associated with that dental health service package.

The system may advantageously be provided with a logistics component to support the user in inventory management of the equipment that is required to perform the actions in the dental hygiene scheme. In many cases, the dental actions included in the dental hygiene scheme require specific equipment or 'general' equipment, such as tooth brushes, having specific characteristics. The dental specialist may during an appointment provide the user with the required information. However, to support the user and allow the user to review the specific characteristics of the equipment needed by the user, the dental specialist may enter these characteristics in a list of equipment in the logistics component. The list for example may specify one or more of the type of toothbrush that is to be used, such as a hard, soft or medium toothbrush or an electric or hand tooth brush, the type of dental floss, the type of mouthwash or dental glue for dental prostheses. Other, more specific equipment may also be entered in the list in the logistics component. The logistics component may additionally also be modifyable by the user to keep track of a quantity of the equipment in the list that is available by the user.

The logistics component may also be configured to communicate, preferably via the communication interface of the system, with a communication interface, such as a website or web-form, of a supplier of the equipment. This is especially advantageous for equipment that is non-standard and therefore difficult to obtain for a user and may for example be performed by having software that is compatible with the dental health service package.

In an embodiment of the system according to the invention, the digital devices may be digital devices associated with the user or digital devices associated with the dental specialist of the user, wherein each of the digital devices is provided with a service application that allows the user or the dental specialist to be automatically authenticated to access the dental health service package of the user in the memory when the service application is started on the digital device.

In an advantageous embodiment, the digital device may be supplied with a service application that simplifies remotely accessing the dental health service package of the user in the memory. The service application may therein be configured for authenticating a specific digital device for access to the dental health service package without requiring the user to supply authentication information. In other words, the service application only requires a user to authenticate the digital device a single time, after which the service application, preferably upon starting, automatically connects with the dental health service package in the (remotely located) memory of the system.

In an embodiment of the system according to the invention, the system may be provided with multiple authentication levels associated with different types of users.

By applying multiple authentication levels, the system may in an easy manner be configured for different users. This may include limiting the number of dental health service packages that an individual user may access, based on the type of user and may also or alternatively include limiting the read/write authorisation that is provided to a specific user and/or a specific group of users. This may for example include allowing dental specialists access to multiple uniquely mapped dental health service packages of users that are his patients or clients, and may additionally or alternatively also include limiting the read/write authentication within a uniquely mapped dental health service packages. The latter may for example be used to allow a dental specialist to modify all information in the uniquely mapped dental health service packages, whereas it allows a user to only modify some parts, such as inventory lists, within his or her own uniquely mapped dental health service package. Preferably, the system is provided with at least one super-user having full access rights to the system, wherein the superuser configures the system and determines access rights to the system. Furthermore, the system is preferably configured to restrict access to a dental health service package of a user to a limited number of users, for example three user constituting the superuser, the dental specialist associated with the user and the user. The system may also be configured to allow a user to indicate to the system which users may have access to the system. In an elaboration, such access may be provided by allowing a user to scan a code, for example a QR-code, of a dental specialist that is allowed access to the dental health service package of the user, which is relayed in the system to allow the dental specialist access to the system. The system may also be configured to only allow registered users to obtain access rights. Registration may for example be limited to dental specialists and their patients, which can be coupled in the system.

In an embodiment of the system according to the invention, the system may comprise an encryption component for, after retrieval of information from the memory, encrypting information from the dental health service package before sending the information over a communication network to a digital device of a user or a dental specialist associated with that user, wherein the digital device is provided with a service application that is configured for decrypting the encrypted information.

In order to provide additional (digital) security to the system, the system may be provided with an encryption component that encrypts all data that is sent from the communication interface of the system to (external) digital devices. The digital devices in turn will in this embodiment be provided with a service application that is configured for decrypting the data that is received from the system to enable the data and/or information contained therein to be provided to the user, for example on a display of the digital device.

In an embodiment, the system may additionally comprise a logbook module that is configured for storing user-input containing information on one or more of feedback on dental actions performed by the user and nutrition intake by the user, such as sugar intake, preferably in the form of a nutrition history.

Having a logbook function in the system allows both the user and the dental specialist to keep track of the actions to be performed (user) and the actions that were performed (dental specialist). This is for example useful when providing a dental awareness and guidance program in which the user is guided and taught about a good dental health behaviour for the particular set of teeth of the user.

In an embodiment of the system according to the invention, the system may additionally comprise a user interface, preferably a graphical user interface (GUI), that is configured for creating and/or displaying a dental health service package of the user.

The invention also relates to a computer program product comprising computer-executable instructions for performing a method according to the invention, when the program is run on a computer.

The computer program product according to the invention provides the same effects and advantages as the method and the system according to the invention as described above.

Further advantages, features and details of the invention are elucidated on the basis of preferred embodiments thereof, and reference is made to the accompanying drawings, in which:
- Figure 1 shows a schematic example of the method steps according to the invention;
- Figure 2 shows a schematic view of an example of a system according to the invention; and
- Figure 3 shows a schematic view of a second example of a system according to the invention.

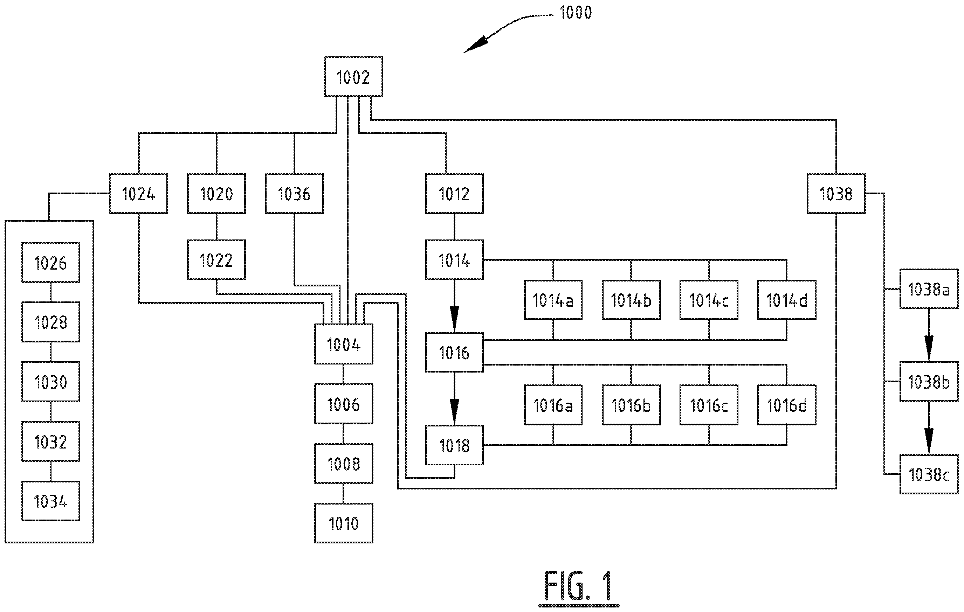

A schematic example of the method according to the invention including various embodiments is shown in figure 1. In the example, method 1000 includes creating 1002 a uniquely mapped dental health service package of a user. After creating 1002 the uniquely mapped dental health service package of a user, the method also comprises a step of storing 1004 the uniquely mapped dental health service package of a user and subsequently accessing 1006 the memory using a digital device and obtaining 1008 the uniquely mapped dental health service package of a user. Accessing 1006 may be performed directly after the steps of creating 1002 and storing 1004, can be performed at a later time or a combination thereof. The accessing 1006 in most cases will be performed when a specific action on the set of teeth of the user is required, such as performing dental care or in case of an emergency. In this example, the method further comprises providing 1010 the user with the uniquely mapped dental health service package. This is performed by providing a visualisation of the data using a display of the digital device the user is using to obtain access to the memory.

Creating 1002 the uniquely mapped dental health service package may include several steps that may be executed separately or in conjunction with each other. The method according to this example first of all includes the step of performing 1012 a visual inspection by a dental specialist of the set of teeth of the user and also creating and analysing 1014 a dental image of the set of teeth of the user. The creating and analysing step 1014 may include one or more actions, including the steps of determining 1014a, 1014b, 1014c the number, position and lay-out of the teeth and molars in the set of teeth of that particular user. In addition, in this example it includes determining 1014d a state of dental care of the identified teeth and molars. These steps may also be performed in conjunction with or as part of the performing step 1012.

By performing these steps, preferably during an appointment at the dental specialist, the dental specialist is able to determine the state of the set of teeth, which he can add to the dental image in order to obtain a complete overview of the current state of the set of teeth. The analysis that is made forms the basis for the method step of creating 1016 a dental scheme by selecting dental care actions that the user needs to perform to maintain or improve the state of dental health of his set of teeth. The specific dental care actions are provided by the dental specialist from his expertise and are adapted to the specific set of teeth of the user, after which they are added to the dental scheme. This includes the step of storing 1016a, 1016b, 1016c, 1016d information on, amongst others, sequence, intensity and duration of brushing and interdental cleaning that is to be performed by the user on his set of teeth. Each of these parameters is determined by the dental specialist and is uniquely connected to the set of teeth of that particular user, thus uniquely mapping the actions for his set of teeth.

Creating 1002 the uniquely mapped dental health service package in this example also includes providing 1020 and adding 1022 information on dental corrections and dental repairs, which include information on crowns of teeth, dental fillings, oral implants and dental prosthesis to the dental image. This is preferably performed by adding a digital, interactive layer on the dental image, which can be accessed during accessing and/or displaying the dental health service package. The information may not only include indicating that a repair is performed, but may also provide more specific information on the repair, such as the duration, the materials used in the repair and/or the specific state of the tooth or molar before and after the repair. Any irregularities may also be noted.

Furthermore, the step of creating 1002 the uniquely mapped dental health service package in this example also includes the step of providing 1024 a dental emergency service. The service includes the steps of contacting 1026 a dental emergency service, for example a weekend dentist, due to a problem that suddenly has occurred in the set of teeth and accessing 1028 the information in the uniquely mapped dental health service package to provide these to the weekend dentist. Supplying the information may be performed by sending 1030 the information over a communication network or may be performed by authorising 1032 the dental specialist and allowing 1034 him to via a digital device access the uniquely mapped dental health service package of the user to render him direct assistance.

Additionally, in the example the method comprises the step of providing 1036 a uniquely mapped list of equipment the user requires for performing the dental scheme.

The method according to this example also includes the step of sending push-notifications to the digital device of a user as part of the dental hygiene scheme in the uniquely mapped dental health service package. Such push notifications may include appointment reminders 1038a, notifications of dental care actions to be taken 1038b and notification to update 1038c the dental specialist with regard to the dental care actions that have been performed and/or the results thereof.

In practice, the method according to the invention would preferably start during a visit of a user to his or her dental specialist. During the visual inspection of the set of teeth of the user by the dental specialist, the dental specialist notes the specific lay-out and condition of the set of teeth, which he, preferably using a dental image, codifies in the dental health service package. Furthermore, during the visual inspection and based on his expertise, the dental specialist selects the dental care actions that are to be performed by the user for an optimal care of the set of teeth. This includes providing a sequence of brushing and optionally includes (interdental) cleaning, that the user needs to perform, which is visualised in the dental health service package, which may be provided as an app on a mobile device of the user. As such, the user can access the expert information specifically adapted to his set of teeth at the moment he needs it, i.e. the moment he performs the dental care actions.

In a preferred embodiment, the dental care actions selected by the dental specialist are also discussed with the user. This increases the awareness of the user on the dental care actions and allows the user to provide specific information that may be useful for the dental health service package and the dental care actions contained therein.

Furthermore, the dental specialist preferably also provides information on dental repairs and/or modifications he or she makes to the set of teeth in the dental health service package, therewith providing a historical overview that is accessible by both dental specialist as well as the user.

As a result, the dental health service package allows the expertise of the dental specialist that is applied to a unique set of teeth of the user to be accessible at any given time by that user. Moreover, the dental health service package provides a guide for the user on the dental care actions, such as the sequence of brushing/cleaning of his teeth, to achieve an optimal dental care for his set of teeth. Furthermore, in practice the app may also provide additional features such as a guidance program for children and young adults, i.e up to around 18 years of age, which stimulates their dental care routine and increases awareness on dental health care.

In use of the method of providing a uniquely dental health service package to a user including unique dental information, the dental specialist starts a dental health service system from a terminal, preferably a computer in the dental practice, and logs in to the system using his credentials as specialist. After logging in to the dental health service system, the dental specialist has access to the dental health service packages of patients that are linked to his practice and is provided with the option to create new dental health service packages for a (new) patient. The options for adapting information of patients and/or the option to create a new dental health service package that is uniquely coupled to a specific patient is provided to the dental specialist by means of a dashboard.

Upon creating of a new dental health service package that is uniquely coupled to a specific patient, the dental specialist is provided with a dental image of a standard set of teeth. In a next step, the dental specialist visually inspects the set of teeth of the user to identify unique aspects of the set of teeth of the user. This includes identifying the teeth and/or molars that are missing and removes the teeth/molars from the standard dental image to create the dental image that is uniquely adapted to the set of teeth of the user. This step may alternatively be provided by making a dental image, for example an X-ray image of the set of teeth, which the system uses to automatically update the standard dental image.

After modifying the dental image to reflect the unique set of teeth of the user, the dental health service package assigns an identifier, for example a number, to each of the teeth and/or molars in the set of teeth. Additional information, such as information of modifications is entered and linked to associated teeth or molars. As such, the system creates a set of numbered items to which information can be added by the dental specialist. This information for example includes any modifications made to a specific numbered item and/or the state of maintenance thereof.

In a subsequent step, the dental health service package creates different views of the set of teeth, each view showing a specific dental care quadrant, such as the outside of the lower jaw and the inside of the lower jaw, and requests the dental specialist to assign dental care actions to each of the numbered items in each of the quadrants. This includes for example brushing and/or flossing.

As a result, a uniquely coupled dental health service package is provided that is assigned to a memory from which the dental health service package is made accessible for the user. A user of whom a uniquely coupled dental health service package is created, downloads an application to a digital device that provides an access portal to the dental health service package that is uniquely coupled to that user. In order to get access, the user must activate the application by scanning a digital access code, for example a QR code, of the dental specialist. The user subsequently can access the information that is uniquely adapted to his set of teeth. The dental health service package provides a user interface that is accessible from a digital device having a display and can be stored in (i.e. downloaded to) a memory of the digital device or can be remotely accessed by the digital device. The dental health service package provides real-time information to the user on his unique set of teeth, by displaying the dental image (for his set of teeth) and provides the brushing instructions that are specifically adapted to the unique set of teeth of the user. Additionally, the dental health service package may provide an overview of all the equipment that is required for the dental actions that are displayed by the dental health service package. The dental health service package also provides the user direct and real-time access to a dental modification passport, which contains an overview of (at least part of) the modifications to the set of teeth that have been made. This may include an overview of dental prosthesis, implants and/or other information including information that is relevant for a dentist in case of (dental) emergency.

The dental health package may additionally be provided with several other functionalities, which include shopping to allow a user to in the application order the dental health equipment that is required for the dental health actions and/or logging and communicating nutrition information and/or providing push notifications.

The logging and communicating nutrition information of the dental health service package requires a user to supply nutrition intake information including the amount and/or frequency of nutrition intake to the package, which subsequently stores the information in the memory and/or communicates the information to the dentist.

The push notifications may be generated by the dental health service package based on calculations of the dental health care actions to be performed at a specific time and/or scheduled appointments with the dental specialist. The push notifications may also comprise a motivational message to perform the dental care actions and/or may comprise an invitation to provide feedback. This may for example take the form of a push notification that requests the user on whether a dental care action was performed, and provides feedback based on the user response. In case of a confirmation of performance, a subsequent push notification is sent containing a feedback option for the user to provide feedback. The push notifications may for example be used to assist a user in reflecting on the (results of the) action that was (to be) performed.

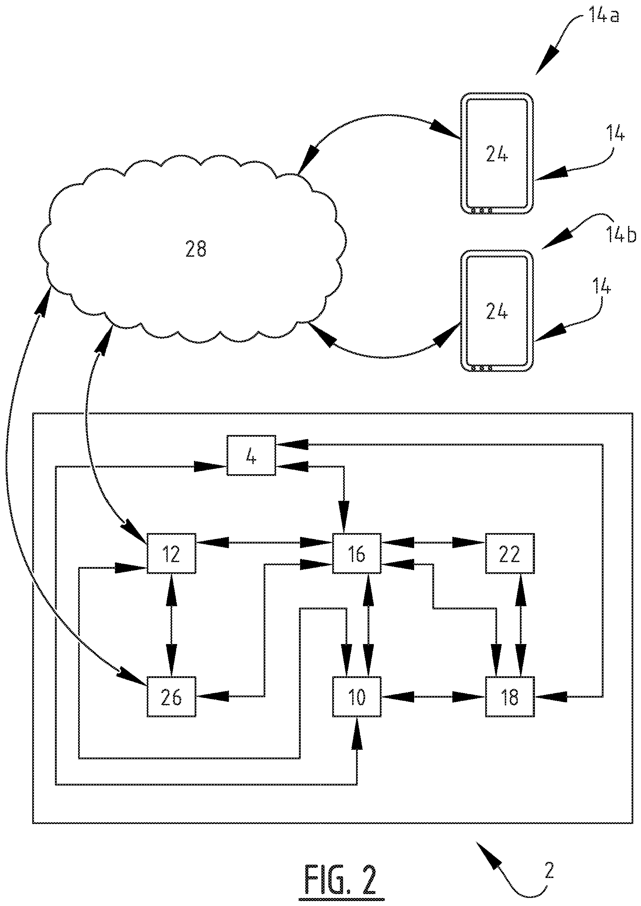

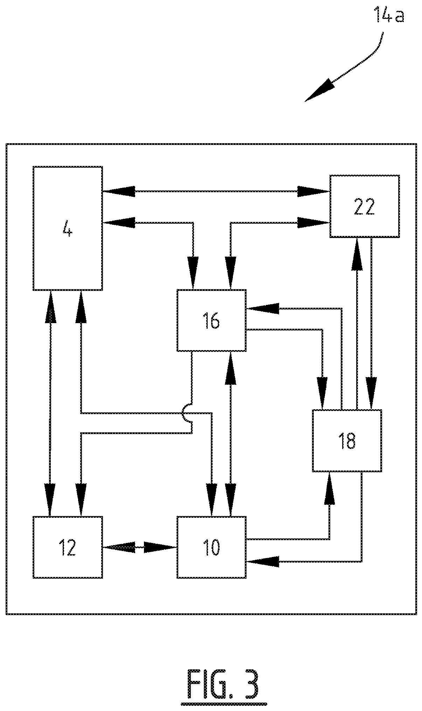

An example of system 2 according to the invention is provided in figures 2 and 3. System 2 (figure 2) includes a memory 4 for storing a number of uniquely mapped dental health service packages 6 of users and an authentication component 10 that are configured to communicate with each other. Communication interface 12 is configured to communicate with memory 4 via authentication component 10, all of which are controlled by processor 16 of system 2. System 2 in this example furthermore includes user interface 22 that is operatively connected to logistics component 18 for storing a list of equipment. A user 8 can access memory 4 using digital device 14, 14a, which connects over communication network 28 with communication interface 12 of system 2. After successful authentication by authentication component 10, which authentication action is controlled by processor 16, user 8 can access his uniquely mapped dental health service package 6 in memory 4 and display it on a display of digital device 14a. In this example, service application 24 and encryption component 26 encrypt and/or decrypt information sent via communication network 28.

Furthermore, memory 4 of system 2 can also be accessed by a dental specialist via digital device 14b in a similar fashion as with user 8. Digital device 14b will sent an access request, which is encrypted by service application 24, to communication interface 12 via communication network 28. Encryption component 26 decrypts the received message and is configured to relay the request to authentication component 10. After successful authentication, the dental specialist can access the uniquely mapped dental health service package 6 of user 8 via digital device 14b and perform the necessary actions.

In a second example (see figure 3), system 2 is a substantially stand-alone system 2. In this example, the uniquely mapped dental health service package 6 of user 8 is provided on digital device 14a of user 8. System 2, which is incorporated in digital device 14a, comprises memory 4, which may be part of the memory of digital device 14a, and processor 16, which is the processor of digital device 14a. Furthermore, system 2 includes user interface 22, for example a touch screen or (touch) buttons, which is operatively connected with logistics component 18. In addition, user interface 22 is connected with memory 4, allowing user 8 direct access to his uniquely mapped dental health service package 6. Communication component 12 and authentication component 10 are in this example primarily used for sending and receiving information regarding uniquely mapped dental health service package 6, which may include updates from or to the dental specialist.

During operation, a dental specialist logs in to the dental health service system using a terminal and supplies his dental specialist credentials, which form part of an authorization request sent to the system. If the supplied credentials are valid, the system displays a dental specialist user interface on a display of the terminal. The user interface comprises a dashboard in which different uniquely coupled dental health service packages to which the dental specialist has access, are displayed. In addition, the dental health service system displays the option for the dental specialist to create a new dental health service package for a patient.

Upon choosing to create a new dental health service package, the system is configured to opens a standard dental health service file that is to be adapted by the dental specialist to create a uniquely coupled dental health service package.

The new standard dental health service package is subsequently displayed on the display including a message to the dental specialist to provide identification information of a user to which the dental health service package is to be coupled. A subsequent step is receiving and processing, by the dental health service system, the identification information supplied by the dental specialist to the dental health service package by coupling the user information to the dental health service package, therewith providing a unique coupling between the user and the dental health service package.

After processing and adding to the dental health service package the user information received by the dental health service system, the method comprises displaying a standard dental image on the display, preferably in addition to displaying a request to the dental specialist for adapting the set of teeth to the specific set of teeth of the user. Modifications can be supplied to the system by means of user input, in this case input of the dental specialist. Each input is processed in the dental health service system and subsequently an adapted dental image is sent to the display.

Alternatively, the system may be configured for receiving a digital image, for example an X-ray image of the unique set of teeth of the user, comparing the X-ray image with the standard dental image and adapting the standard dental image to match with the X-ray image. The automatically updated dental image, which is than uniquely coupled to the set of teeth of the user, is subsequently displayed by the dental health system on the display.

After modification of the set of teeth, the dental health service system stores the adapted dental image as part of the dental health service package to the memory. After adapting the dental image to match the unique set of teeth of the user to which the dental health service package is coupled, the dental health system is configured for adding identifiers, preferably numbers, to each of the individual teeth and molars in the set of teeth and subsequently displaying an overview of the uniquely coupled dental image of the user including the identifiers on the display.

Preferably, the overview is accompanied by a request to the dental specialist for adding other unique information. The dental specialist is, by means of the request, prompted to select for each of the identifiers appropriate dental care actions and/or dental care materials from a list of dental care actions and/or from a list of dental care materials. After entering the information on the set of teeth of the patient, the dental specialist provides a command to the dental health service system, for example by clicking a user interface button, to save and close the dental health service package, upon which the system is configured to save the uniquely coupled dental health service package to the memory from which it is accessible by a user using a digital device.

The method further comprises downloading by a user a dental health service application to a digital device associated with that user. In a subsequent step, the method comprises coupling the application on the digital device to the uniquely coupled dental health service package of the user. Preferably, the coupling comprises scanning, by the user using the digital device, a code that is configured to authorize the application to access the memory for retrieving the uniquely coupled dental health service package coupled to that user. After authorising and retrieving the uniquely coupled dental health service package by the application on the digital device, the application is configured to display a user interface on a display of the digital device that includes an overview of dental care actions for each of the individual identifiers (i.e. each individual tooth and molar) that is to be performed by the user. The application is further configured to, in response to a user action, start a timer indicating a period that a user is to perform the dental care actions on each tooth or molar as well as the sequence in which the dental actions are to be performed, wherein the dental care actions are the dental care actions as supplied by the dental specialist and stored by the dental health service system in the memory.

Additionally, the application may be configured to regularly send an update request to the memory for retrieving updates on the uniquely coupled dental health service package. Furthermore, the application may be configured to generate push-notifications, wherein a push-notification is based on a comparison of the dental care actions and the current time as provided by the digital device, and wherein the generated push-notification is supplied to the user by displaying the notification on the display and/or providing another signalling action. The push notifications may also be generated by the dental health service package based a comparison of a scheduled appointment with the dental specialist and the current time and/or date.

The present invention is by no means limited to the above described preferred embodiments thereof. The rights sought are defined by the following claims within the scope of which many modifications can be envisaged.

## Claims

1. Method for providing a uniquely mapped dental health service package to a user by providing uniquely mapped dental information of the user on a digital device (14a, 14b), the method comprising:
- creating a dental health service package comprising a dental scheme for an individual user, the dental scheme comprising dental care actions to be performed by the user on its set of teeth, wherein the dental health service package is uniquely mapped for a set of teeth of the individual user in that is adapted to the set of teeth of the user, and wherein the dental health service package comprises electronically encoded information regarding the set of teeth of the individual user,
- wherein creating the dental health service package for the individual user comprises:
- providing a dental image of a standard set of teeth;
- identifying unique aspects of the set of teeth of the user, which includes identifying and removing teeth and/or molars that are missing from the standard dental image to create the dental image that is uniquely adapted to the set of teeth of the user; and
- assigning an identifier to each of the teeth and/or molars in the set of teeth;
- storing the uniquely mapped dental health service package in a memory (4) by a dental specialist;
- accessing the memory by the user using a digital device (14a, 14b);
- obtaining the uniquely mapped dental health service package of the user by the user using the digital device; and
- providing to the user the uniquely mapped dental health service package of the user, wherein the step of providing the uniquely mapped dental health service package to the user comprises:
- displaying on a digital device a representation, preferably a visualisation, of the set of teeth of the user including the identifiers and the individualised dental care actions for each of the teeth in the set of teeth by providing a visual indicator that displays one or more of the sequence, duration and/or intensity of the brushing for each of the teeth;
- sending a push-notification to the user with a notification of a dental hygiene action to be performed;
- supplying by the user feedback information to the dental health information package in response to a dental health action performed by the user;
- storing the feedback information in a logbook of the dental health service package; and
- sending by the dental health service package a feedback notification adapted to the information supplied by the user;
wherein the dental health service package is further configured to allow the user to send some or all information on its teeth to the dental specialist or the dental institution that is to perform dental emergency assistance and/or to allow the user to authorize a dental specialist or a dental institution access to the uniquely mapped dental health service package of the user.

2. Method according to claim 1, wherein the dental health service package also provides the user direct and real-time access to a dental modification passport, which contains an overview of (at least part of) the modifications to the set of teeth that have been made, wherein the dental modification passport preferably includes an overview of dental prosthesis, implants and/or other information including information that is relevant for a dentist in case of (dental) emergency; and/or wherein the information may comprise a screen shot of the dental image and/or a screen shot of the information on the tooth/molar that is currently causing the emergency.

3. Method according to claim 1 or 2, wherein creating a dental health service package for an individual user further comprises:
- performing at least one of:
- visual inspection by a dental specialist of the set of teeth of the user; and
- creating and analysing a dental image of the set of teeth of the user;
- based on the analysis of the dental image and/or the visual inspection, creating the dental scheme by selecting dental care actions to be performed by the user on their set of teeth; and
- adding personal information of the user to the dental health service package for identifying the user.

4. Method according to claim 1, 2 or 3, wherein analysing the dental image comprises one or more of the following actions:
- determining a number of teeth and/or molars and/or oral implants in the set of teeth of the user;
- determining a position of the teeth and/or molars and/or oral implants in a jaw bone of the user and/or a position of the teeth and/or molars relative to each other;
- determining a position of any gaps or openings due to missing teeth and/or molars in a jaw bone of the user and/or a position of the gaps or openings due to missing teeth and/or molars relative to other teeth;
- determining a state of dental care of the set of teeth of the user, including gums, dental nerves and/or dental roots associated with the set of teeth, the dental care state comprising determining the presence of one or more of dental plaque, crowns of a tooth, dental prostheses, dental fillings, oral implants, gingivitis and/or periodontitis and/or comparing a dental care state, especially a risk profile of the set of teeth, with a benchmark including one or more of a plaque index, a DPSI-index, a smoking index and a bleeding index.

5. Method according to any one of the preceding claims, when dependent on claim 3, wherein selecting dental care actions to be performed by the user comprises:
- storing first information comprising a sequence of brushing and/or cleaning of the teeth of the user; and/or
- storing second information comprising a duration and/or intensity of brushing and/or cleaning to be performed by the user for each of teeth and/or molars;
wherein the first and/or second information is uniquely mapped for the user.

6. Method according to any one of the preceding claims, when dependent on claim 3, wherein selecting dental care actions to be performed by the user comprises:
- storing third information comprising the sequence of interdental cleaning of the teeth of the user; and/or
- storing fourth information comprising the duration and/or intensity of interdental cleaning to be performed by the user for each of the spaces between the teeth;
wherein the third and/or fourth information is uniquely mapped for the user.

7. Method according to claim 5 or 6, wherein at least one of the first, second, third and fourth stored information is provided to the user on a screen of the digital device, wherein providing the information comprises displaying the dental image of the user and a visual indicator that displays one or more of the sequence, duration and/or intensity of the brushing, preferably for each of the teeth, and/or the interdental cleaning, preferably the interdental cleaning of the spaces between the teeth, wherein the dental image and the visual indicator are preferably incorporated in a video feed that is uniquely mapped to the user, such as a brushing or interdental cleaning movie, that provides a uniquely mapped guideline for dental care of the set of teeth of the user.

8. Method according to any one of the preceding claims, the method further comprising:
- providing information on dental corrections and dental repairs, which include information on crowns of teeth, dental fillings, oral implants and dental prosthesis, preferably during the visual inspection of the set of teeth of the user; and
- adding the information on dental corrections and dental repairs to a dental image of the set of teeth of the user, preferably in the form of a digital, interactive layer on the dental image, and/or wherein the method additionally comprises providing a list of equipment that is uniquely mapped to the user and that is required for performing the dental care actions in the dental scheme, wherein the list preferably is provided by a logistics module of the dental health service package; and/or
the method further comprising:
- supplying to the dental health information package by the user one or more of:
- instructions on dental health actions to be performed based on user-actions comprising intake of nutrition;
- information on nutrition intake comprising amount and/or frequency of nutrition intake of the user, such as sugar intake;
- storing the respectively supplied instructions, information on nutrition intake comprising amount and/or frequency of nutrition intake in a logbook of the dental health service package.

9. Method according to any one of the preceding claims, further comprising sending uniquely mapped push-notifications to the digital device of the user as part of the dental scheme, wherein the push-notifications include one or more of the following notifications:
- a reminder of an appointment with the dental specialist;
- a notification to fill out a questionnaire and/or update a status of the dental scheme actions to be performed by the user, such as providing information on the performed dental actions or the dental actions to be performed, wherein the questionnaire and/or the status update is coupled to the dental health service package and therewith is uniquely mapped to that user;
- a random feedback notification, which notification is adapted to the information provided by the user in the status update or questionnaire, wherein the feedback notification preferably includes a motivational message.

10. Method according to claim 9, when dependent on claim 8, the method comprising providing an integrated dental awareness and guidance schedule that is uniquely mapped to the user, the user preferably being a child or young adult having an age of 20 years or less, preferably 18 years or less, the schedule comprising the steps of:
- based on the dental image and/or visual inspection of the set of teeth of the user, providing a risk analysis on the sensitivity of the set of teeth for tooth decay;
- drafting a guidance schedule for the user, the guidance schedule including:
- the dental health actions to be performed;
- a date and frequency of feedback moments;
- performing the steps according to claim 8;
- performing the step according to claim 9;

11. System for providing a uniquely mapped dental health service package to a user, the system comprising:
- a memory (4) for storing a number of uniquely mapped dental health service packages of users;
- an authentication component (10) that is configured for authenticating access of individual users to one or more uniquely mapped dental health service packages of users that are stored in the memory;
- a communication interface (12) that is configured for sending and/or receiving information to digital devices (14a, 14b), wherein the information comprises one or more of the following: dental health service packages or information forming part thereof and access and/or modification requests; and
- at least one processor (16) for processing and/or controlling at least one of the memory, the authentication component and/or the communication interface,
wherein the at least one processor is configured for executing the method according to any one of the claims 1 - 10.

12. System according to claim 11, wherein the information contains a dental modification passport, which contains an overview of (at least part of) the modifications to the set of teeth that have been made, and which preferably includes an overview of dental prosthesis, implants and/or other information including information that is relevant for a dentist in case of (dental) emergency; and/or
wherein the information may comprise a screen shot of the dental image and/or a screen shot of the information on the tooth/molar that is currently causing the emergency.

13. System according to claim 11 or 12, the system additionally comprising a logistics component that is configured for one or more of:
- storing a list of equipment required for performing the dental hygiene scheme of a dental health service package of the user;
- providing a user interface for allowing the user or the dental specialist associated with a dental health service package to modify the list of equipment that is associated with that dental health service package
- providing access and/or purchase history to a webshop having dental care equipment;
- storing and/or displaying an instruction video;
- receiving and sending feedback notifications;
- registration of functionality and/or registration of functionality use;
- registration of statistical information, the statistical information being accessible by the user and/or the dental specialist.

14. System according to any one of the claims 11 - 13, wherein the digital devices are digital devices associated with the user or digital devices associated with the dental specialist of the user, wherein each of the digital devices is provided with a service application that allows the user or the dental specialist to be automatically authenticated to access the dental health service package of the user in the memory when the service application is started on the digital device, and/or wherein the system is provided with multiple authentication levels associated with different types of users, and/or wherein the system comprising an encryption component for, after retrieval of information from the memory, encrypting information from the dental health service package before sending the information over a communication network to a digital device of a user or a dental specialist associated with that user, wherein the digital device is provided with a service application that is configured for decrypting the encrypted information, and/or wherein the system additionally comprising a logbook module that is configured for storing user-input containing information on one or more of:
- feedback on dental actions performed by the user;
- nutrition intake by the user, such as sugar intake, including amount and/or frequency of nutrition intake, the nutrition intake preferably being in the form of a nutrition history.

15. A computer program product comprising computer-executable instructions for performing a method according to any one of the claims 1 - 10, when the program is run on computers.

## Patentansprüche

1. Verfahren zum Bereitstellen eines eindeutig dargestellten Zahngesundheitsdienstpakets an einen Benutzer durch Bereitstellen eindeutig dargestellter Zahninformationen des Benutzers auf einer digitalen Vorrichtung (14a, 14b), das Verfahren umfassend:
- Erstellen eines Zahngesundheitsdienstpakets, umfassend ein Zahnschema für einen einzelnen Benutzer, das Zahnschema umfassend Zahnpflegehandlungen, die von dem Benutzer an seinem Gebiss durchzuführen sind, wobei das Zahngesundheitsdienstpaket eindeutig für ein Gebiss des einzelnen Benutzers dargestellt ist, indem es an das Gebiss des Benutzers angepasst ist, und wobei das Zahngesundheitsdienstpaket elektronisch codierte Informationen hinsichtlich des Gebisses des einzelnen Benutzers umfasst,
- wobei das Erstellen des Zahngesundheitsdienstpakets für den einzelnen Benutzer umfasst:
- Bereitstellen eines Zahnbildes eines Standardgebisses;
- Identifizieren von eindeutigen Gesichtspunkten des Gebisses des Benutzers, die das Identifizieren und Entfernen von Zähnen und/oder Molaren, die bei dem Standardzahnbild fehlen, einschließt, um das Zahnbild zu erzeugen, das eindeutig an das Gebiss des Benutzers angepasst ist; und
- Zuweisen einer Kennung zu jedem der Zähne und/oder Molaren in dem Gebiss;
- Speichern des eindeutig dargestellten Zahngesundheitsdienstpakets in einem Speicher (4) durch einen Zahnspezialisten;
- Zugreifen auf den Speicher durch den Benutzer unter Verwendung einer digitalen Vorrichtung (14a, 14b);
- Erhalten des eindeutig dargestellten Zahngesundheitsdienstpakets des Benutzers durch den Benutzer unter Verwendung der digitalen Vorrichtung; und
- Bereitstellen des eindeutig dargestellten Zahngesundheitsdienstpakets des Benutzers an den Benutzer, wobei der Schritt des Bereitstellens des eindeutig dargestellten Zahngesundheitsdienstpakets an den Benutzer umfasst:
- Anzeigen einer Darstellung, vorzugsweise einer Visualisierung, des Gebisses des Benutzers, die die Kennungen und die individualisierten Zahnpflegehandlungen für jeden der Zähne in dem Gebiss durch Bereitstellen einer visuellen Anzeige, die eine oder mehrere von der Abfolge, der Dauer und/oder der Intensität des Bürstens für jeden der Zähne auf einer digitalen Vorrichtung anzeigt, einschließt;
- Senden einer Push-Benachrichtigung an den Benutzer mit einer Benachrichtigung einer Zahnhygienehandlung, die durchzuführen ist;
- Zuführen von Benutzerrückmeldungsinformationen an das Zahngesundheitsinformationspaket als Reaktion auf eine von dem Benutzer durchgeführte Zahnhygienehandlung;
- Speichern der Rückmeldungsinformationen in einem Logbuch des Zahngesundheitsdienstpakets; und
- Senden einer Rückmeldungsbenachrichtigung, die an die von dem Benutzer zugeführten Informationen angepasst ist, durch das Zahngesundheitsdienstpaket;
wobei das Zahngesundheitsdienstpaket ferner konfiguriert ist, um es dem Benutzer zu ermöglichen, einige oder alle Informationen über seine Zähne an den Zahnspezialisten oder das zahnärztliche Institut, das Zahnnotfallunterstützung leisten soll, zu senden und/oder um es dem Benutzer zu ermöglichen, einen Zahnspezialisten oder ein zahnärztliches Institut zu autorisieren, auf das eindeutig dargestellte Zahngesundheitsdienstpaket des Benutzers zuzugreifen.

2. Verfahren nach Anspruch 1, wobei das Zahngesundheitsdienstpaket dem Benutzer auch direkten und Echtzeit-Zugriff auf einen Zahnmodifikationspass bereitstellt, der eine Übersicht über (mindestens einen Teil) der an dem Gebiss getätigten Modifikationen enthält, wobei der Zahnmodifikationspass vorzugsweise einen Überblick über Zahnprothese, Implantate und/oder andere Informationen, einschließlich Informationen, die für einen Zahnarzt im Fall von (zahnärztlichen) Notfällen relevant sind, einschließt; und/oder wobei die Informationen einen Screenshot des Zahnbildes und/oder einen Screenshot der Informationen über den Zahn/Molar, der aktuell den Notfall verursacht, umfassen können.

3. Verfahren nach Anspruch 1 oder 2, wobei das Erstellen eines Zahngesundheitsdienstpakets für einen einzelnen Benutzer ferner umfasst:
- Durchführen von mindestens einem von:
- visueller Inspektion des Gebisses des Benutzers durch einen Zahnspezialisten; und
- Erzeugen und Analysieren eines Zahnbildes des Gebisses des Benutzers;
- basierend auf der Analyse des Zahnbildes und/oder der visuellen Inspektion, Erstellen des Zahnschemas durch Auswählen von Zahnpflegehandlungen, die von dem Benutzer auf seinem Gebiss durchzuführen sind; und
- Hinzufügen von persönlichen Informationen des Benutzers zu dem Zahngesundheitsdienstpaket zum Identifizieren des Benutzers.

4. Verfahren nach Anspruch 1, 2 oder 3, wobei das Analysieren des Zahnbildes eine oder mehrere der folgenden Handlungen umfasst:
- Bestimmen einer Anzahl von Zähnen und/oder Molaren und/oder oralen Implantaten in dem Gebiss des Benutzers;
- Bestimmen einer Position der Zähne und/oder Molaren und/oder oralen Implantate in einem Kieferknochen des Benutzers und/oder einer Position der Zähne und/oder Molaren in Bezug aufeinander;
- Bestimmen einer Position von beliebigen Lücken oder Öffnungen aufgrund fehlender Zähne und/oder Molaren in einem Kieferknochen des Benutzers und/oder einer Position der Lücken oder Öffnungen aufgrund fehlender Zähne und/oder Molaren in Bezug auf andere Zähne;
- Bestimmen eines Zahnpflegezustands des Gebisses des Benutzers, einschließlich des Zahnfleischs, Zahnnerven und/oder Zahnwurzeln, die dem Gebiss zugeordnet sind, der Zahnpflegezustand umfassend das Bestimmen der Anwesenheit einer oder mehrerer von Zahnbelag, Kronen eines Zahns, Zahnprothesen, Zahnfüllungen, oralen Implantaten, Gingivitis und/oder Parodontitis und/oder Vergleichen eines Zahnpflegezustands, insbesondere eines Risikoprofils des Gebisses, mit einem Bezugswert, der einen oder mehrere von einem Plaque-Index, einem DPSI-Index, einem Rauchindex und einem Blutungsindex einschließt.

5. Verfahren nach einem der vorstehenden Ansprüche, wenn er von Anspruch 3 abhängig ist, wobei das Auswählen von Zahnpflegehandlungen, die von dem Benutzer durchzuführen sind, umfasst:
- Speichern erster Informationen, umfassend eine Abfolge von Bürsten und/oder Reinigen der Zähne des Benutzers; und/oder
- Speichern von zweiten Informationen, umfassend eine Dauer und/oder eine Intensität von Bürsten und/oder Reinigen, die von dem Benutzer für jeden der Zähne und/oder Molaren durchzuführen sind;
wobei die ersten und/oder die zweiten Informationen für den Benutzer eindeutig dargestellt sind.

6. Verfahren nach einem der vorstehenden Ansprüche, wenn er von Anspruch 3 abhängig ist, wobei das Auswählen von Zahnpflegehandlungen, die von dem Benutzer durchzuführen sind, umfasst:
- Speichern dritter Informationen, umfassend die Abfolge von Zahnzwischenraumreinigen der Zähne des Benutzers; und/oder
- Speichern vierter Informationen, umfassend die Dauer und/oder die Intensität von Zahnzwischenraumreinigen, die von dem Benutzer für jeden der Zwischenräume zwischen den Zähnen durchzuführen sind;
wobei die dritten und/oder die vierten Informationen für den Benutzer eindeutig dargestellt sind.

7. Verfahren nach Anspruch 5 oder 6, wobei dem Benutzer mindestens eine der ersten, der zweiten, der dritten und der vierten gespeicherten Informationen auf einem Bildschirm der digitalen Vorrichtung bereitgestellt wird, wobei das Bereitstellen der Informationen das Anzeigen des Zahnbildes des Benutzers und einer visuellen Anzeige umfasst, die eine oder mehrere von der Abfolge, der Dauer und/oder der Intensität des Bürsten, vorzugsweise für jeden der Zähne, und/oder die Zahnzwischenraumreinigung, vorzugsweise die Zahnzwischenraumreinigung der Räume zwischen den Zähnen, anzeigt, wobei das Zahnbild und die visuelle Anzeige vorzugsweise in einem Videofeed integriert sind, der für den Benutzer eindeutig dargestellt ist, wie ein Bürsten- oder ein Zahnzwischenraumreinigungsfilm, der eine eindeutig dargestellte Richtlinie für die Zahnpflege des Gebisses des Benutzers bereitstellt.

8. Verfahren nach einem der vorstehenden Ansprüche, das Verfahren ferner umfassend:
- Bereitstellen von Informationen über Zahnkorrekturen und Zahnreparaturen, die Informationen über Kronen auf Zähnen, Zahnfüllungen, orale Implantate und Zahnprothese einschließen, vorzugsweise während der visuellen Inspektion des Gebisses des Benutzers; und
- Hinzufügen der Informationen über Zahnkorrekturen und Zahnreparaturen zu einem Zahnbild des Gebisses des Benutzers, vorzugsweise in Form einer digitalen, interaktiven Schicht auf dem Zahnbild, und/oder wobei das Verfahren zusätzlich das Bereitstellen einer Ausrüstungsliste umfasst, die für den Benutzer eindeutig dargestellt ist, und die zum Durchführen der Zahnpflegehandlungen in dem Zahnschema erforderlich ist, wobei die Liste vorzugsweise durch ein Logistikmodul des Zahngesundheitsdienstpakets bereitgestellt wird; und/oder das Verfahren ferner umfassend:
- Zuführen von dem Benutzer an das Zahngesundheitsinformationspaket eines oder mehrerer von:
- Anweisungen über Zahngesundheitshandlungen, die basierend auf Benutzerhandlungen, umfassend Aufnahme von Nahrung, durchzuführen sind;
- Informationen über die Nahrungsaufnahme, umfassend die Menge und/oder die Häufigkeit der Nahrungsaufnahme des Benutzers, wie Zuckeraufnahme;
- Speichern der jeweils zugeführten Anweisungen, Informationen über die Nahrungsaufnahme, umfassend die Menge und/oder die Häufigkeit der Nahrungsaufnahme in einem Logbuch des Zahngesundheitsdienstpakets.

9. Verfahren nach einem der vorstehenden Ansprüche, ferner umfassend das Senden von eindeutig dargestellten Push-Benachrichtigungen an die digitale Vorrichtung des Benutzers als Teil des Zahnschemas, wobei die Push-Benachrichtigungen eine oder mehrere der folgenden Benachrichtigungen einschließen:
- eine Erinnerung an einen Termin mit dem Zahnspezialisten;
- eine Benachrichtigung, um einen Fragebogen auszufüllen und/oder einen Status der Zahnschemahandlungen, die von dem Benutzer durchzuführen sind, zu aktualisieren, wie das Bereitstellen von Informationen über die durchgeführten Zahnhandlungen oder die Zahnhandlungen, die durchzuführen sind, wobei der Fragebogen und/oder die Statusaktualisierung mit dem Zahngesundheitsdienstpaket gekoppelt sind und damit eindeutig für den Benutzer dargestellt sind;
- eine zufällige Rückmeldungsbenachrichtigung, wobei die Benachrichtigung an die Informationen, die von dem Benutzer in der Statusaktualisierung oder dem Fragebogen bereitgestellt sind, angepasst ist, wobei die Rückmeldungsbenachrichtigung vorzugsweise eine Motivationsnachricht einschließt.

10. Verfahren nach Anspruch 9, wenn er von Anspruch 8 abhängig ist, das Verfahren umfassend das Bereitstellen eines integrierten Zahnbewusstseins- und Führungszeitplans, der für den Benutzer eindeutig dargestellt ist, wobei der Benutzer vorzugsweise ein Kind oder junger Erwachsener in einem Alter von 20 Jahren oder weniger, vorzugsweise 18 Jahren oder weniger, ist, der Zeitplan umfassend die Schritte:
- basierend auf dem Zahnbild und/oder der visuellen Inspektion des Gebisses des Benutzers, das Bereitstellen einer Risikoanalyse auf der Empfindlichkeit des Gebisses für Karies;
- Entwerfen eines Führungszeitplans für den Benutzer, der Führungszeitplan einschließend:
- die Zahngesundheitshandlungen, die durchzuführen sind;
- ein Datum und eine Häufigkeit von Rückmeldungszeitpunkten;
- Durchführen der Schritte nach Anspruch 8;
- Durchführen des Schritts nach Anspruch 9;

11. System zum Bereitstellen eines eindeutig dargestellten Zahngesundheitsdienstpakets an einen Benutzer, das System umfassend:
- einen Speicher (4) zum Speichern einer Anzahl von eindeutig dargestellten Zahngesundheitsdienstpaketen von Benutzern;
- eine Authentifizierungskomponente (10), die zum Authentifizieren des Zugriffs einzelner Benutzer auf ein oder mehrere eindeutig dargestellte Zahngesundheitsdienstpakete von Benutzern, die in dem Speicher gespeichert sind, konfiguriert ist;
- eine Kommunikationsschnittstelle (12), die zum Senden und/oder Empfangen von Informationen an digitale Vorrichtungen (14a, 14b) konfiguriert ist, wobei die Informationen eines oder mehrere der Folgenden umfassen: Zahngesundheitsdienstpakete oder Informationen, die einen Teil davon und Zugriffs- und/oder Modifikationsanforderungen bilden; und
- mindestens einen Prozessor (16) zum Verarbeiten und/oder Steuern von mindestens einem von dem Speicher, der Authentifizierungskomponente und/oder der Kommunikationsschnittstelle, wobei der mindestens eine Prozessor zum Ausführen des Verfahrens nach einem der Ansprüche 1 bis 10 konfiguriert ist.

12. System nach Anspruch 11, wobei die Informationen einen Zahnmodifikationspass enthalten, der eine Übersicht über (mindestens einen Teil) der an dem Gebiss getätigten Modifikationen enthält, und der vorzugsweise einen Überblick über Zahnprothese, Implantate und/oder andere Informationen, einschließlich Informationen, die für einen Zahnarzt im Fall von (zahnärztlichen) Notfällen relevant sind, einschließt; und/oder
und/oder wobei die Informationen einen Screenshot des Zahnbildes und/oder einen Screenshot der Informationen über den Zahn/Molar, der aktuell den Notfall verursacht, umfassen können.

13. System nach Anspruch 11 oder 12, das System zusätzlich umfassend eine Logistikkomponente, die konfiguriert ist für eines oder mehrere von:
- Speichern einer Ausrüstungsliste, die zum Durchführen des Zahnhygieneschemas eines Zahngesundheitsdienstpakets des Benutzers erforderlich ist;
- Bereitstellen einer Benutzerschnittstelle, die es ermöglicht, dass der Benutzer oder der Zahnspezialist, der einem Zahngesundheitsdienstpaket zugeordnet ist, die Ausrüstungsliste modifiziert, die diesem Zahngesundheitsdienstpaket zugeordnet ist
- Bereitstellen des Zugriffs und/oder einer Kaufhistorie an einen Webshop mit Zahnpflegeausrüstung;
- Speichern und/oder Anzeigen eines Anweisungsvideos;
- Empfangen und Senden von Rückmeldungsbenachrichtigungen;
- Registrieren der Funktionalität und/oder Registrierung der Verwendungsfunktionalität;
- Registrieren statistischer Informationen, wobei die statistischen Informationen durch den Benutzer und/oder den Zahnspezialisten zugänglich sind.

14. System nach einem der Ansprüche 11 bis 13, wobei die digitalen Vorrichtungen digitale Vorrichtungen sind, die dem Benutzer oder digitalen Vorrichtungen, die dem Zahnspezialisten des Benutzers zugeordnet sind, zugeordnet sind, wobei jede der digitalen Vorrichtungen mit einer Dienstanwendung versehen ist, die es dem Benutzer oder dem Zahnspezialisten ermöglicht, automatisch authentifiziert zu werden, um auf das Zahngesundheitsdienstpaket des Benutzers in dem Speicher zuzugreifen, wenn die Dienstanwendung auf der digitalen Vorrichtung gestartet wird, und/oder wobei das System mit mehreren Authentifizierungsstufen versehen ist, die verschiedenen Arten von Benutzern zugeordnet sind, und/oder wobei das System eine Verschlüsselungskomponente zum Verschlüsseln, nach Abruf von Informationen aus dem Speicher, von Informationen aus dem Zahngesundheitsdienstpaket vor dem Senden von Informationen über ein Kommunikationsnetzwerk an eine digitale Vorrichtung eines Benutzers oder eines Zahnspezialisten, der diesem Benutzer zugeordnet ist, umfasst, wobei die digitale Vorrichtung mit einer Dienstanwendung versehen ist, die zum Entschlüsseln der verschlüsselten Informationen konfiguriert ist, und/oder wobei das System zusätzlich ein Logbuchmodul umfasst, das zum Speichern von Benutzereingaben konfiguriert ist, enthaltend Informationen über eine oder mehrere einer:
- Rückmeldung zu Zahnhandlungen, die von dem Benutzer durchgeführt werden;
- Nahrungsaufnahme durch den Benutzer, wie Zuckeraufnahme, einschließlich Menge und/oder Häufigkeit der Nahrungsaufnahme, wobei die Nahrungsaufnahme vorzugsweise in Form einer Nahrungshistorie vorliegt.

15. Computerprogrammprodukt, umfassend computerausführbare Anweisungen zum Durchführen eines Verfahrens nach einem der Ansprüche 1 bis 10, wenn das Programm auf Computern ausgeführt wird.

## Revendications

1. Procédé permettant de fournir un ensemble de services de santé dentaire à correspondance unique à un utilisateur en fournissant des informations dentaires à correspondance unique de l'utilisateur sur un dispositif numérique (14a, 14b), le procédé comprenant :
- la création d'un ensemble de services de santé dentaire comprenant un schéma dentaire pour un utilisateur individuel, le schéma dentaire comprenant des actions de soins dentaires à mettre en oeuvre par l'utilisateur sur sa dentition, l'ensemble de services de santé dentaire étant mis en correspondance de façon unique pour une dentition de l'utilisateur individuel en ce qu'il est adapté à la dentition de l'utilisateur, et l'ensemble de services de santé dentaire comprenant des informations encodées électroniquement concernant la dentition de l'utilisateur individuel,
- dans lequel la création de l'ensemble de services de santé dentaire pour l'utilisateur individuel comprend :
- la fourniture d'une image dentaire d'une dentition standard ;
- l'identification d'aspects uniques de la dentition de l'utilisateur, qui comporte l'identification et le retrait de dents et/ou de molaires qui sont manquantes de l'image dentaire standard pour créer l'image dentaire qui est adaptée de façon unique à la dentition de l'utilisateur ; et
- l'attribution d'un identificateur à chacune des dents et/ou molaires dans la dentition ;
- le stockage de l'ensemble de services de santé dentaire à correspondance unique dans une mémoire (4) par un spécialiste dentaire ;
- l'accès à la mémoire par l'utilisateur à l'aide d'un dispositif numérique (14a, 14b) ;
- l'obtention de l'ensemble de services de santé dentaire à correspondance unique de l'utilisateur par l'utilisateur à l'aide du dispositif numérique ; et
- la fourniture à l'utilisateur, de l'ensemble de services de santé dentaire à correspondance unique de l'utilisateur, l'étape de fourniture de l'ensemble de services de santé dentaire à correspondance unique à l'utilisateur comprenant :
- l'affichage sur un dispositif numérique d'une représentation, de préférence une visualisation, de la dentition de l'utilisateur comportant les identificateurs et les actions de soins dentaires individualisées pour chacune des dents dans la dentition en fournissant un indicateur visuel qui affiche une ou plusieurs parmi la séquence, la durée et/ou l'intensité du brossage pour chacune des dents ;
- l'envoi d'une notification push à l'utilisateur avec une notification d'une action d'hygiène dentaire à mettre en oeuvre ;
- la remise par l'utilisateur d'informations de rétroaction à l'ensemble d'informations de santé dentaire en réponse à une action de santé dentaire mise en oeuvre par l'utilisateur. ;
- le stockage des informations de rétroaction dans un journal de bord de l'ensemble de services de santé dentaire ; et
- l'envoi par l'ensemble de services de santé dentaire d'une notification de rétroaction adaptée aux informations remises par l'utilisateur ;
l'ensemble de services de santé dentaire étant configuré en outre pour permettre à l'utilisateur d'envoyer certaines ou toutes les informations concernant ses dents au spécialiste dentaire à l'institution dentaire qui doit mettre en oeuvre une assistance dentaire d'urgence et/ou pour permettre à l'utilisateur d'autoriser un spécialiste dentaire ou une institution dentaire à accéder à l'ensemble de services de santé dentaire à correspondance unique de l'utilisateur.

2. Procédé selon la revendication 1, dans lequel l'ensemble de services de santé dentaire fournit également à l'utilisateur un accès direct et en temps réel à un passeport de modification dentaire, qui contient une vue d'ensemble (d'au moins une partie) des modifications qui ont été apportées à la dentition, le passeport de modification dentaire comportant de préférence une vue d'ensemble de prothèse dentaire, d'implants et/ou d'autres informations comportant des informations qui sont pertinentes pour un dentiste en cas d'urgence (dentaire) ; et/ou dans lequel les informations peuvent comprendre une capture d'écran de l'image dentaire et/ou une capture d'écran des informations concernant la dent/molaire qui provoque actuellement l'urgence.

3. Procédé selon la revendication 1 ou 2, dans lequel la création d'un ensemble de services de santé dentaire pour un utilisateur individuel comprend en outre :
- la mise en oeuvre d'au moins l'un parmi :
- une inspection visuelle par un spécialiste dentaire de la dentition de l'utilisateur ; et
- la création et l'analyse d'une image dentaire de la dentition de l'utilisateur ;
- en fonction de l'analyse de l'image dentaire et/ou de l'inspection visuelle, la création du schéma dentaire en sélectionnant des actions de soins dentaires à mettre en oeuvre par l'utilisateur sur sa dentition ; et
- l'ajout d'informations personnelles de l'utilisateur à l'ensemble de services de santé dentaire pour identifier l'utilisateur.

4. Procédé selon la revendication 1, 2 ou 3, dans lequel l'analyse de l'image dentaire comprend une ou plusieurs des actions suivantes :
- détermination d'un nombre de dents et/ou molaires et/ou implants oraux dans la dentition de l'utilisateur ;
- détermination d'une position des dents et/ou molaires et/ou implants oraux dans un os de mâchoire de l'utilisateur et/ou d'une position des dents et/ou molaires les unes par rapport aux autres ;
- détermination d'une position d'espaces ou ouvertures éventuels en raison de dents et/ou molaires manquantes dans un os de mâchoire de l'utilisateur et/ou d'une position des espaces ou ouvertures en raison de dents et/ou molaires manquantes par rapport à d'autres dents ;
- détermination d'un état de soins dentaires de la dentition de l'utilisateur, y compris gencives, nerfs dentaires et/ou racines dentaires associés à la dentition, l'état de soins dentaires comprenant la détermination de la présence d'un ou plusieurs parmi plaque dentaire, couronnes d'une dent, prothèses dentaires, obturations dentaires, implants oraux, gingivite et/ou parodontite et/ou la comparaison d'un état de soins dentaires, spécialement un profil de risque de la dentition, à un point de référence comportant un ou plusieurs parmi un indice de plaque, un indice DPSI, un indice de tabagisme et un indice de saignement.

5. Procédé selon l'une quelconque des revendications précédentes, prise en dépendance de la revendication 3, dans lequel la sélection des actions de soins dentaires à mettre en oeuvre par l'utilisateur comprend :
- le stockage de premières informations comprenant une séquence de brossage et/ou nettoyage des dents de l'utilisateur ; et/ou
- le stockage de deuxièmes informations comprenant une durée et/ou une intensité de brossage et/ou nettoyage à mettre en oeuvre par l'utilisateur pour chacune de dents et/ou molaires ;
les premières et/ou deuxièmes informations étant à correspondance unique pour l'utilisateur.

6. Procédé selon l'une quelconque des revendications précédentes, prise en dépendance de la revendication 3, dans lequel la sélection des actions de soins dentaires à mettre en oeuvre par l'utilisateur comprend :
- le stockage de troisièmes informations comprenant la séquence de nettoyage interdentaire des dents de l'utilisateur ; et/ou
- le stockage de quatrièmes informations comprenant la durée et/ou l'intensité de nettoyage interdentaire à mettre en oeuvre par l'utilisateur pour chacun des espaces entre les dents ;
les troisièmes et/ou quatrièmes informations étant à correspondance unique pour l'utilisateur.

7. Procédé selon la revendication 5 ou 6, dans lequel au moins l'une des premières, deuxièmes, troisièmes et quatrièmes informations stockées sont fournies à l'utilisateur sur un écran du dispositif numérique, la fourniture des informations comprenant l'affichage de l'image dentaire de l'utilisateur et d'un indicateur visuel qui affiche une ou plusieurs parmi la séquence, la durée et/ou l'intensité du brossage, de préférence pour chacune des dents, et/ou le nettoyage interdentaire, de préférence le nettoyage interdentaire des espaces entre les dents, l'image dentaire et l'indicateur visuel étant de préférence incorporés dans un flux vidéo qui est mis en correspondance de façon unique pour l'utilisateur, tel qu'une vidéo de brossage ou de nettoyage interdentaire, qui fournit une recommandation à correspondance unique pour des soins dentaires de la dentition de l'utilisateur.

8. Procédé selon l'une quelconque des revendications précédentes, le procédé comprenant en outre :
- la fourniture d'informations concernant des corrections dentaires et réparations dentaires, qui comportent des informations concernant des couronnes de dents, des obturations dentaires, des implants oraux et une prothèse dentaire, de préférence pendant l'inspection visuelle de la dentition de l'utilisateur ; et
- l'ajout des informations concernant les corrections dentaires et réparations dentaires à une image dentaire de la dentition de l'utilisateur, de préférence sous la forme d'une couche interactive numérique sur l'image dentaire, et/ou le procédé comprenant en outre la fourniture d'une liste de matériel qui est à correspondance unique pour l'utilisateur et qui est requis pour mettre en oeuvre les actions de soins dentaires dans le schéma dentaire, la liste étant fournie de préférence par un module logistique de l'ensemble de services de santé dentaire ; et/ou
le procédé comprenant en outre :
- la remise à l'ensemble d'informations de santé dentaire par l'utilisateur d'une ou plusieurs parmi :
- des instructions concernant des actions de santé dentaire à mettre en oeuvre en fonction d'actions de l'utilisateur comprenant l'apport nutritionnel ;
- des informations concernant l'apport nutritionnel comprenant la quantité et/ou la fréquence d'apport nutritionnel de l'utilisateur, tel que l'apport de sucre ;
- le stockage des instructions, des informations concernant l'apport nutritionnel comprenant la quantité et/ou la fréquence d'apport nutritionnel, respectivement remises, dans un journal de bord de l'ensemble de services de santé dentaire.

9. Procédé selon l'une quelconque des revendications précédentes, comprenant en outre l'envoi de notifications push à correspondance unique au dispositif numérique de l'utilisateur dans le cadre du schéma dentaire, les notifications push comportant une ou plusieurs des notifications suivantes :
- un rappel d'un rendez-vous avec le spécialiste dentaire ;
- une notification pour remplir un questionnaire et/ou mettre à jour un statut des actions de schéma dentaire à mettre en oeuvre par l'utilisateur, tel que la fourniture d'informations concernant les actions dentaires mises en oeuvre ou les actions dentaires à mettre en oeuvre, le questionnaire et/ou la mise à jour de statut étant couplés à l'ensemble de services de santé dentaire et avec celui-ci étant à correspondance unique pour cet utilisateur ;
- une notification de rétroaction aléatoire, cette notification étant adaptée aux informations fournies par l'utilisateur dans la mise à jour de statut ou le questionnaire, la notification de rétroaction comportant de préférence un message de motivation.

10. Procédé selon la revendication 9, prise en dépendance de la revendication 8, le procédé comprenant la fourniture d'un programme intégré de sensibilisation et conseils dentaires qui est à correspondance unique pour l'utilisateur, l'utilisateur étant de préférence un enfant ou jeune adulte ayant un âge de 20 ans ou moins, de préférence 18 ans ou moins, le programme comprenant les étapes consistant à :
- en fonction de l'image dentaire et/ou de l'inspection visuelle de la dentition de l'utilisateur, fournir une analyse de risque sur la sensibilité de la dentition à la carie dentaire ;
- rédiger un programme de conseils pour l'utilisateur, le programme de conseils comprenant :
- les actions de santé dentaire à mettre en oeuvre ;
- une date et une fréquence de moments de rétroaction ;
- mettre en oeuvre les étapes selon la revendication 8 ;
- mettre en oeuvre l'étape selon la revendication 9 ;

11. Système permettant de fournir un ensemble de services de santé dentaire à correspondance unique à un utilisateur, le système comprenant :
- une mémoire (4) permettant de stocker un certain nombre d'ensembles de services de santé dentaire à correspondance unique d'utilisateurs ;
- un composant d'authentification (10) qui est configuré pour authentifier un accès d'utilisateurs individuels à un ou plusieurs ensembles de services de santé dentaire à correspondance unique d'utilisateurs, qui sont stockés dans la mémoire ;
- une interface de communication (12) qui est configurée pour envoyer et/ou recevoir des informations à des dispositifs numériques (14a, 14b), les informations comprenant un ou plusieurs de ce qui suit : ensembles de services de santé dentaire ou informations en faisant partie et demandes d'accès et/ou de modification ; et
- au moins un processeur (16) permettant de traiter et/ou de commander au moins l'un parmi la mémoire, le composant d'authentification et/ou l'interface de communication, l'au moins un processeur étant configuré pour exécuter le procédé selon l'une quelconque des revendications 1 à 10.

12. Système selon la revendication 11, dans lequel les informations contiennent un passeport de modification dentaire, qui contient une vue d'ensemble (d'au moins une partie) des modifications qui ont été apportées à la dentition, et qui comporte de préférence une vue d'ensemble de prothèse dentaire, d'implants et/ou d'autres informations comportant des informations qui sont pertinentes pour un dentiste en cas d'urgence (dentaire) ; et/ou
dans lequel les informations peuvent comprendre une capture d'écran de l'image dentaire et/ou une capture d'écran des informations concernant la dent/molaire qui provoque actuellement l'urgence.

13. Système selon la revendication 11 ou 12, le système comprenant en outre un composant logistique qui est configuré pour un ou plusieurs parmi :
- le stockage d'une liste de matériel requis pour mettre en oeuvre le schéma d'hygiène dentaire d'un ensemble de services de santé dentaire de l'utilisateur ;
- la fourniture d'une interface utilisateur pour permettre à l'utilisateur ou au spécialiste dentaire associé à un ensemble de services de santé dentaire de modifier la liste de matériel qui est associée à cet ensemble de services de santé dentaire
- la fourniture d'un accès et/ou d'un historique d'achat sur une boutique en ligne ayant du matériel de soins dentaires ;
- le stockage et/ou l'affichage d'une vidéo d'instruction ;
- la réception et l'envoi de notifications de rétroaction ;
- l'enregistrement de fonctionnalité et/ou l'enregistrement d'utilisation de fonctionnalité ;
- l'enregistrement d'informations statistiques, les informations statistiques étant accessibles par l'utilisateur et/ou le spécialiste dentaire.

14. Système selon l'une quelconque des revendications 11 à 13, dans lequel les dispositifs numériques sont des dispositifs numériques associés à l'utilisateur ou des dispositifs numériques associés au spécialiste dentaire de l'utilisateur, chacun des dispositifs numériques étant pourvu d'une application de service qui permet à l'utilisateur ou au spécialiste dentaire d'être automatiquement authentifié pour accéder à l'ensemble de services de santé dentaire de l'utilisateur dans la mémoire lorsque l'application de service est démarrée sur le dispositif numérique, et/ou le système étant pourvu de multiples niveaux d'authentification associés à différents types d'utilisateurs, et/ou le système comprenant un composant de chiffrement permettant, après récupération d'informations en mémoire, de chiffrer les informations provenant de l'ensemble de services de santé dentaire avant l'envoi des informations sur un réseau de communication vers un dispositif numérique d'un utilisateur ou d'un spécialiste dentaire associé à cet utilisateur, le dispositif numérique étant pourvu d'une application de service qui est configurée pour déchiffrer les informations chiffrées, et/ou le système comprenant en outre un module journal de bord qui est configuré pour stocker une entrée d'utilisateur contenant des informations concernant un ou plusieurs parmi :
- une rétroaction concernant des actions dentaires mises en oeuvre par l'utilisateur ;
- un apport nutritionnel par l'utilisateur, tel qu'un apport en sucre, comportant la quantité et/ou la fréquence d'apport nutritionnel, l'apport nutritionnel étant de préférence sous la forme d'un historique de nutrition.

15. Produit programme d'ordinateur comprenant des instructions exécutables par ordinateur permettant de mettre en oeuvre un procédé selon l'une quelconque des revendications 1 à 10, lorsque le programme est exécuté sur des ordinateurs.
